# FASCICULE DE BREVET EUROPEEN

(11) **EP 2 184 065 B1**
(45) Date de publication et mention de la délivrance du brevet: **20.07.2011**
(21) Numéro de dépôt: 09290841.7
(22) Date de dépôt: 06.11.2009
(51) Int. Cl.: A61K 31/55, A61K 49/00

(54) **Ivabradine et son utilisation dans une méthode de diagnostic utilisant la méthode d'angiographie coronaire par tomodensitométrie multicoupe**
Ivabradin und seine Verwendung in einer Diagnosemethode unter Einsatz der koronaren Angiographie durch Mehrschicht-Computertomographie
Ivabradine and its use in a method of diagnosis using coronary angiography with multi-slice computed tomography

(30) Priorité: 07.11.2008 FR 0806225
(43) Date de publication de la demande: 12.05.2010
(73) Titulaire: Les Laboratoires Servier, 92284 Suresnes Cedex (FR)
(72) Inventeur: Lerebours-Pigeonniere, Guy, 92300 Levallois Perret (FR); Dubost-Brama, Ariane, 75015 Paris (FR); Fleurinck, Carmen, 9820 Schelderode (BE)
(74) Mandataire: Giudicelli, Cathy

(56) Documents cités:
- EP-A- 1 695 965
- EP-A- 1 944 031
- MANZ M ET AL: "A SINGLE INTRAVENOUS DOSE OF IVABRADINE, A NOVEL IF INHIBITOR, LOWERS HEART RATE BUT DOES NOT DEPRESS LEFT VENTRICULAR FUNCTION IN PATIENTS WITH LEFT VENTRICULARR DYSFUNCTION" CARDIOLOGY, KARGER, BASEL, CH, vol. 100, no. 3, 1 novembre 2003 (2003-11-01), pages 149-155, XP009059994 ISSN: 0008-6312
- "IVABRADINE HYDROCHLORIDE ANTIANGINAL HCN (LF CURRENT) BLOCKER" DRUGS OF THE FUTURE, PROUS SCIENCE, ES, vol. 28, no. 7, 1 janvier 2003 (2003-01-01), pages 652-658, XP009052804 ISSN: 0377-8282
- PANNU H K ET AL: "[beta]-blockers for cardiac CT: A primer for the radiologist" AMERICAN JOURNAL OF ROENTGENOLOGY 200606 US, vol. 186, no. 6 SUPPL. A, juin 2006 (2006-06), pages S341-S345, XP002531243 ISSN: 0361-803X
- SHAPIRO ET AL: "Efficacy of pre-scan beta-blockade and impact of heart rate on image quality in patients undergoing coronary multidetector computed tomography angiography", EUROPEAN JOURNAL OF RADIOLOGY, vol. 66 , - April 2008 (2008-04), pages 37-41,
- DE GRAAF ET AL: "Evaluation of Contraindications and Efficacy of Oral Beta Blockade Before Computed Tomographic Coronary Angiography", THE AMERICAN JOURNAL OF CARDIOLOGY, vol. 105, no. 6 , - 15 March 2010 (2010-03-15), pages 767-772,
- BAX ET AL: "A randomized double blind trial on the efficacy and safety of a single intravenous bolus of ivabradine versus placebo for heart rate control during coronary CT angiography", EUROPEAN HEART JOURNAL, vol. 31 , - 2010, page 151,
- GUARICCI ET AL: "Incremental value and safety of oral ivabradine for heart rate reduction in computed tomography coronary angiography", INTERNATIONAL JOURNAL OF CARDIOLOGY, - 23 November 2010 (2010-11-23),

## Description

La présente invention concerne l'utilisation de l'ivabradine ou 3-{3-[{[(7S)-3,4-diméthoxybicyclo[4.2.0]octa-1,3,5-trién-7-yl]-méthyl}-(méthyl)-amino]-propyl}-7,8-diméthoxy-1,3,4,5-tétrahydro-2*H*-3-benzazépin-2-one de formule (I): 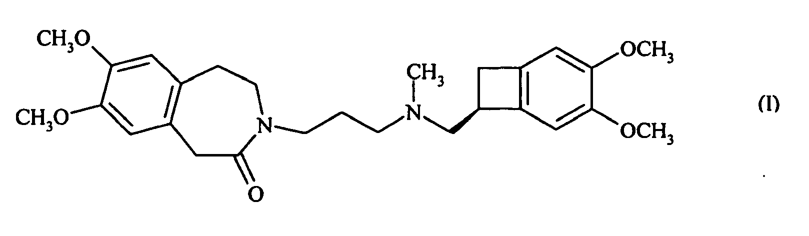 ainsi que ses sels d'addition à un acide pharmaceutiquement acceptable et les hydrates desdits sels d'addition, dans une méthode de diagnostic utilisant la méthode d'angiographie coronaire par tomodensitométrie multicoupe.

L'ivabradine, ainsi que ses sels d'addition à un acide pharmaceutiquement acceptable, et plus particulièrement son chlorhydrate, et les hydrates desdits sels d'addition, possèdent des propriétés pharmacologiques et thérapeutiques très intéressantes.
Ils réduisent de façon directe et sélective l'activité pacemaker cardiaque, ce qui leur confère des propriétés chronotropes négatives (réduction de la fréquence cardiaque), sans affecter la pression artérielle, ce qui permet de les envisager dans le traitement, la prévention et l'amélioration du pronostic de différentes maladies cardio-vasculaires liées à l'ischémie myocardique telles que l'angine de poitrine, l'infarctus du myocarde et dans l'insuffisance cardiaque chronique

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0 534 859.

La demanderesse a présentement trouvé que l'ivabradine et ses sels d'addition, plus particulièrement son chlorhydrate, possédaient des propriétés intéressantes permettant leur utilisation dans une méthode de diagnostic utilisant la méthode d'angiographie coronaire par tomodensitométrie multicoupe.

L'angiographie coronaire par tomodensitométrie multicoupe, ou MSCT-CA (MultiSlice Computed Tomography Coronary Angiography), encore appelée MDCT-CA (MultiDetector Computed Tomography Coronary Angiography), est une technique rapide et non invasive permettant d'examiner les artères coronaires et de détecter par imagerie une maladie coronarienne, notamment le rétrécissement (sténose) ou l'obstruction des coronaires, ainsi que d'évaluer l'anatomie et la perméabilité des vaisseaux, et de caractériser les plaques athéromateuses au niveau tissulaire. Cette méthode évite le recours à la technique classique d'angiographie par cathétérisme cardiaque, qui, de par son caractère invasif, comporte des risques.

Dans la méthode d'angiographie coronaire par tomodensitométrie multicoupe, un produit de contraste iodé est injecté au patient pour opacifier la lumière des coronaires. L'acquisition des images se fait ensuite par radiation aux rayons X à l'aide d'un scanner multi-barrettes (c'est-à-dire multi-détecteurs).

Les artères coronaires sont des vaisseaux de petit calibre, tortueux et en mouvement rapide, donc difficiles à imager. Par conséquent, une résolution élevée à la fois spatiale et temporelle est requise pour les analyser correctement. Elle est d'autant meilleure que le nombre de barrettes est élevé.

Le scanner multi-barrettes a en général de 4 à 64 détecteurs. Les scanners les plus récents sont munis de 64 détecteurs, et parfois d'une double source de rayons X, qui augmente la capacité de résolution temporelle de la technique.

D'autre part, en raison des artéfacts de mouvements, la qualité des images est affectée par une fréquence cardiaque élevée.

PANNU H K ET AL (AMERICAN JOURNAL OF ROENTGENOLOGY, vol. 186, no. 6 SUPPL. A, pages S341-S345) et SHAPIRO ET AL (EUROPEAN JOURNAL OF RADIOLOGY, vol. 66 , pages 37- 41) décrivent un protocole d'étude clinique pour l'administration du métoprolol, un bêta-bloquant, chez les patients soumis à une angiographie coronaire par tomodensitométrie multicoupe pour améliorer la qualité des images obtenues en baissant la fréquence cardiaque.

La demanderesse a présentement trouvé que l'ivabradine était capable de baisser la fréquence cardiaque de façon précoce. Cette propriété permet d'envisager l'utilisation de l'ivabradine chez les patients ayant une fréquence cardiaque élevée soumis à une angiographie coronaire par tomodensitométrie multicoupe pour améliorer la qualité des images obtenues. De plus, une réduction de l'irradiation pourrait être envisagée par la réduction de la fréquence cardiaque.

Ainsi, la présente invention concerne l'utilisation de l'ivabradine, de ses sels d'addition à un acide pharmaceutiquement acceptable et des hydrates desdits sels, pour l'obtention de compositions utilisées dans une méthode de diagnostic utilisant la méthode d'angiographie coronaire par tomodensitométrie multicoupe.

Les compositions sont sous une forme convenant à une administration par voie orale ou intraveineuse, préférentiellement par voie intraveineuse.

La posologie utile varie selon la fréquence cardiaque au repos de la personne à examiner et s'échelonne de 2 à 20 mg par administration.

L'administration par voie intraveineuse est effectuée en un bolus ou par perfusion.

Par bolus, on entend une administration rapide, d'une durée préférentiellement inférieure à 30 secondes.

Les compositions convenant pour une administration par voie intraveineuse peuvent être sous forme de solution injectable ou de lyophilisat à dissoudre dans un solvant avant administration.
La solution injectable est préférentiellement une solution saline.
La concentration d'ivabradine base dans la solution injectable est préférentiellement comprise entre 1 et 5 mg/ml.
Le pourcentage de principe actif de formule (I) dans la solution injectable est préférentiellement compris entre 0.1% et 0.5% en poids.

Le pourcentage de principe actif de formule (I) dans le lyophilisat est préférentiellement compris entre 10% et 50% en poids.

Les compositions convenant pour une administration par voie orale contiennent, outre l'ivabradine, un de ses sels d'addition à un acide pharmaceutiquement acceptable ou un des hydrates de l'un desdits sels d'addition, un ou plusieurs excipients ou véhicules tels que des diluants, des lubrifiants, des liants, des agents de désintégration, des absorbants, des colorants, des édulcorants.

A titre d'exemple et de manière non limitative, on peut citer :
◆ *pour les diluants* : le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
**◆** *pour les liants* : le silicate d'aluminium et de magnésium, l'amidon, la gélatine, la tragacanthe, la méthylcellulose, la carboxyméthylcellulose de sodium et la polyvinylpyrrolidone (PVP),
◆ *pour les désintégrants* : l'agar, l'acide alginique et son sel de sodium, les mélanges effervescents.

Le pourcentage de principe actif de formule (I) dans la composition pour administration par voie orale est préférentiellement compris entre 3% et 50% en poids.

### EXEMPLE 1 : Etude clinique. Effet d'une administration i.v. du chlorhydrate d'ivabradine sur la fréquence cardiaque chez le volontaire sain.

La fréquence cardiaque au repos (en position allongée) est mesurée à T0. Les sujets reçoivent ensuite un bolus i.v. d'une solution de chlorhydrate d'ivabradine dosée à 16 mg d'ivabradine base (groupe traité, n=8) ou de placebo (groupe témoin, n=2).

La fréquence cardiaque au repos (en position allongée) est mesurée à nouveau à T0 + 30 min.

### Résultats :

Chez les sujets traités par l'ivabradine, la fréquence cardiaque est inférieure de 16% par rapport à la fréquence cardiaque du groupe témoin.

### EXEMPLE 2 : Etude clinique. Effet d'une administration i.v. du chlorhydrate d'ivabradine sur la fréquence cardiaque chez le patient soumis à une angiographie coronaire par tomodensitométrie multicoupe.

Les patients sélectionnés pour cette étude ont une fréquence cardiaque au repos égal ou supérieure à 70 bpm.
La fréquence cardiaque au repos du patient est mesurée à T0.
Les patients ayant une fréquence cardiaque comprise entre 70 bpm et 79 bpm reçoivent un bolus iv d'une solution de chlorhydrate d'ivabradine dosée à 10 mg d'ivabradine base (groupe traité) ou de placebo (groupe témoin).
Les patients ayant une fréquence cardiaque égale ou supérieure à 80 bpm reçoivent un bolus iv d'une solution de chlorhydrate d'ivabradine dosée à 15 mg d'ivabradine base (groupe traité) ou de placebo (groupe témoin).
La fréquence cardiaque au repos est mesurée en continu après l'injection du bolus.
Dès que la fréquence cardiaque est inférieure à 65 bpm, le patient est soumis à l'angiographie coronaire.
Un produit de contraste est injecté au patient. L'acquisition des images se fait ensuite par radiation aux rayons X à l'aide d'un scanner multi-barrettes possédant au moins 64 détecteurs.

### EXEMPLE 3 : Solution injectable dosée à 10 mg/5 ml :

Formule de préparation pour 1000 ampoules dosées à 10 mg d'ivabradine base:

| | |
|---|---|
| Chlorhydrate d'ivabradine | 10.78 g |
| Chlorure de sodium | 45 g |
| Eau injectable | 51 |

Les composants sont mélangés et la solution résultante répartie en 1000 ampoules d'une contenance de 10 ml chacune.

### EXEMPLE 4 : Solution injectable dosée à 15 mg/7.5 ml :

Formule de préparation pour 1000 ampoules dosées à 15 mg d'ivabradine base:

| | |
|---|---|
| Chlorhydrate d'ivabradine | 16.17 g |
| Chlorure de sodium | 67.5 g |
| Eau injectable | 7.51 |

Les composants sont mélangés, la solution résultante répartie en 1000 ampoules d'une contenance de 10 ml chacune.

### EXEMPLE 5 : Lyophilisat pour administration par voie intraveineuse

Les composants de l'Exemple 2 sont mélangés, la solution résultante est répartie en 1000 ampoules d'une contenance de 10 ml chacune, qui sont ensuite lyophilisées.

### EXEMPLE 6 : Composition pour administration par voie orale

| Formule de préparation pour 1000 comprimés dosés à 5 mg d'ivabradine base: | |
|---|---|
| Chlorhydrate d'ivabradine | 5,39 g |
| Amidon de maïs | 20 g |
| Silice colloïdale anhydre | 0,2 g |
| Mannitol | 63,91 g |
| Povidone (PVP) | 10 g |
| Stéarate de magnésium | 0,5 g |

## Revendications

1. Ivabradine ou 3-{3-[{[(7S)-3,4-diméthoxybicyclo[4.2.0]octa-1,3,5-trién-7-yl]-méthyl}-(méthyl)-amino]-propyl}-7,8-diméthoxy-1,3,4,5-tétrahydro-2*H*-3-benzazépin-2-one, ses sels d'addition à un acide pharmaceutiquement acceptable ou les hydrates desdits sels, pour son utilisation dans une méthode de diagnostic utilisant la méthode d'angiographie coronaire par tomodensitométrie multicoupe.

2. Composition contenant de l'ivabradine ou 3-{3-[{[(7S)-3,4-diméthoxybicyclo[4.2.0]octa-1,3,5-trién-7-yl]-méthyl}-(méthyl)-amino]-propyl}-7,8-diméthoxy-1,3,4,5-tétrahydro-2*H*-3-benzazépin-2-one, de ses sels d'addition à un acide pharmaceutiquement acceptable ou des hydrates desdits sels, en combinaison avec un ou plusieurs excipients pharmaceutiquement acceptables, pour son utilisation dans une méthode de diagnostic utilisant la méthode d'angiographie coronaire par tomodensitométrie multicoupe.

3. Composition selon la revendication 2 pour son utilisation selon la revendication 2, **caractérisée en ce qu'**elle convient pour une administration par voie intraveineuse.

4. Composition selon la revendication 3 pour son utilisation selon la revendication 2, sous forme de solution injectable.

5. Composition selon la revendication 2 pour son utilisation selon la revendication 2, **caractérisée en ce qu'**elle convient pour une administration par voie orale.

## Claims

1. Ivabradine, or 3-{3-[{[(7S)-3,4-dimethoxybicyclo[4.2.0]octa-1,3,5-trien-7-yl]methyl}-(methyl)amino]propyl}-7,8-dimethoxy-1,3,4,5-tetrahydro-2*H*-3-benzazepin-2-one, its addition salts with a pharmaceutically acceptable acid or hydrates of said salts, for use in a diagnostic method using the method of coronary angiography by multislice computed tomography.

2. Composition comprising ivabradine, or 3-{3-[{[(7S)-3,4-dimethoxybicyclo[4.2.0]octa-1,3,5-trien-7-yl]methyl}(methyl)amino]propyl}-7,8-dimethoxy-1,3,4,5-tetrahydro-2*H-*3-benzazepin-2-one, its addition salts with a pharmaceutically acceptable acid or hydrates of said salts, in combination with one or more pharmaceutically acceptable excipients, for use in a diagnostic method using the method of coronary angiography by multislice computed tomography.

3. Composition according to claim 2 for use according to claim 2, **characterised in that** it is suitable for administration by the intravenous route.

4. Composition according to claim 3 for use according to claim 2, in the form of an injectable solution.

5. Composition according to claim 2 for use according to claim 2, **characterised in that** it is suitable for administration by the oral route.

## Patentansprüche

1. Ivabradin oder 3-{3-[{[(7S)-3,4-Dimethoxybicyclo[4.2.0]octa-1,3,5-trien-7-yl]-methyl}-(methyl)-amino]-propyl}-7,8-dimethoxy-1,3,4,5-tetrahydro-2*H*-3-benzazepin-2-on, seine Additionssalze mit einer pharmazeutisch annehmbaren Säure oder die Hydrate dieser Salze zur Verwendung bei einer diagnostischen Methode unter Anwendung der Koronarangiographiemethode durch Vielschnitt-Computertomographie.

2. Zubereitung enthaltend Ivabradin oder 3-{3-[{[(7S)-3,4-Dimethoxybicyclo-[4.2.0]octa-1,3,5-trien-7-yl]-methyl}-(methyl)-amino]-propyl}-7,8-dimethoxy-1,3,4,5-tetrahydro-2*H*-3-benzazepin-2-on, seine Additionssalze mit einer pharmazeutisch annehmbaren Säure oder Hydrate dieser Salze, in Kombination mit einem oder mehreren pharmazeutisch annehmbaren Hilfsstoffen zur Verwendung bei einer diagnostischen Methode unter Anwendung der Koronarangiographiemethode durch Vielschnitt-Computertomographie.

3. Zubereitung nach Anspruch 2 zur Verwendung gemäß Anspruch 2, **dadurch gekennzeichnet, dass** sie für die Verabreichung auf intravenösem Wege geeignet ist.

4. Zubereitung nach Anspruch 3 zur Verwendung gemäß Anspruch 2 in Form einer injizierbaren Lösung.

5. Zubereitung nach Anspruch 2 zur Verwendung gemäß Anspruch 2, **dadurch gekennzeichnet, dass** sie für die Verabreichung auf oralem Wege geeignet ist.
